# EUROPÄISCHE PATENTSCHRIFT

(11) **EP 1 099 116 B1**
(45) Veröffentlichungstag und Bekanntmachung des Hinweises auf die Patenterteilung: **02.10.2002**
(21) Anmeldenummer: 99923400.8
(22) Anmeldetag: 29.03.1999
(51) Int. Cl.: G01N 33/68, G01N 33/577

(54) **Verfahren und Testkit zum Nachweis von zentralem Nervengewebe in erhitzten Fleischerzeugnissen**
Method and kit for the detection of central nervous system tissue in heated meat products
Procédé et kit d'essai pour la détection de tissu du système nerveux central dans des produits carnés chauffés

(30) Priorität: 30.03.1998 DE 19814088
(43) Veröffentlichungstag der Anmeldung: 16.05.2001
(73) Patentinhaber: ScheBo Biotech AG, 35394 Giessen (DE)
(72) Erfinder: SCHEEFERS-BORCHEL, Ursula, D-35435 Wettenberg (DE); LÜCKER, Ernst, D-35440 Linden-Forst (DE); EIGENBRODT, Erich, D-35440 Linden (DE); SCHEEFERS, Hans, D-35435 Wettenberg (DE)
(74) Vertreter: Fritzsche, Thomas M., Dr.
(86) Internationale Anmeldenummer: DE9900966
(87) Internationale Veröffentlichungsnummer: WO99050661

(56) Entgegenhaltungen:
- E. LÜCKER ET AL.: "Verfahren zum Nachweis von im Hinblick auf die bovine spongiforme Enzephalopathie (BSE) unerwünschten Zutaten in Fleischerzeugnissen." FLEISCHWIRTSCHAFT, Bd. 77, Nr. 9, 1997, Seiten 836-840, XP002117960 in der Anmeldung erwähnt
- CHEMICAL ABSTRACTS, vol. 84, no. 25, 21. Juni 1976 (1976-06-21) Columbus, Ohio, US; abstract no. 176169, L. F. ENG ET AL.: "Measurement of glial fibrillary acidic protein by a two-site immunoradiometric assay." Seite 255; Spalte 1; XP002117961 & ANAL. BIOCHEM., Bd. 71, Nr. 1, 1976, Seiten 243-259,
- CHEMICAL ABSTRACTS, vol. 102, no. 11, 18. März 1985 (1985-03-18) Columbus, Ohio, US; abstract no. 92303, M. ALBRECHTSEN ET AL.: "Enzyme-linked immunosorbent assay for the human glial fibrillary acidic protein using a mouse monoclonal antibody." Seite 277; Spalte 1; XP002117962 & J. NEUROCHEM., Bd. 44, Nr. 2, 1985, Seiten 560-566,
- CHEMICAL ABSTRACTS, vol. 123, no. 15, 9. Oktober 1995 (1995-10-09) Columbus, Ohio, US; abstract no. 194529, P. M. MARTIN ET AL.: "A direct comparison of GFAP immunocytochemistry and GFAP concentration in various regions of the ethanol-fixed rat and mouse brain." Seite 787; Spalte 1; XP002117963 & J. NEUROSCI. METHODS, Bd. 58, Nr. 1,2, 1995, Seiten 181-192,
- CHEMICAL ABSTRACTS, vol. 120, no. 21, 23. Mai 1994 (1994-05-23) Columbus, Ohio, US; abstract no. 265148, P. A. E. S. SMITT ET AL.: "Tissue fixation methods alter the immunohistochemical demonstrability of neurofilament proteins, synaptophysin and glial fibrillary acidic protein in human cerebellum." Seite 518; Spalte 2; XP002117964 & ACTA HISTOCHEM., Bd. 95, Nr. 1, 1993, Seiten 13-21,
- U. Manzke et al (1996) Pharma. Ind. 58, 837-841

## Beschreibung

Die Erfindung betrifft ein Verfahren zum qualitativen und quantitativen Nachweis von spezifiziertem Risikomaterial, insbesondere zentralem Nervengewebe in Erzeugnissen wie Lebensmitteln oder sonstigen Produkten und ein Testkit zur Durchführung des Verfahrens.

1985 wurde bei Rindern im Vereinigten Königreich eine neue, ausschließlich tödlich verlaufende Erkrankung des zentralen Nervengewebes, die bovine spongiforme Enzephalopathie (BSE), beschrieben. Die Entstehung der BSE wird heute auf die Verfütterung von nicht ausreichend erhitztem Tierkörpermehl von an Scrapie verendeten Schafen zurückgeführt. Nach dem Auftreten einer neuen Variante der Creutzfeld-Jakob-Erkrankung des Menschen (nvCJD) 1995 im Vereinigten Königreich und sich mehrender wissenschaftlicher Hinweise, besteht heute kein Zweifel mehr daran, das es sich bei der nvCJD um 'BSE des Menschen' handelt. Hierbei hat es sich gezeigt, daß der Erreger offenbar durch den Verzehr von Produkten, die von infizierten Tieren stammen, übertragbar ist. Da weiterhin gefunden wurde, daß die Übertragbarkeit an bestimmte Gewebe, insbesondere Hirn und Rückenmark gebunden ist, werden Gewebe, die eine nachweisbares Infektionspotential tragen, heute als "spezifiziertes Risikomaterial" (SRM) bezeichnet. Dazu zählen auch Augen, Mandeln und Milz.

Rechtsvorschriften, wie die Entscheidung der Komission 97/534/EG (Amtsblatt der Europäischen Gemeinschaften Nr. L 1997, 216: 95-98) sehen vor, daß bei allen in der Europäischen Union geschlachteten Rindern, Schafen und Ziegen deratig spezifiziertes, bei der Schlachtung anfallendes Material beschlagnahmt, vom Tierkörper entfernt, getrennt gesammelt, besonders gekennzeichnet und auch getrennt von den übrigen Konfiskaten beseitigt werden muß. Die daraus entstehenden Kosten werden allein in Deutschland mehr als eine Milliarde DM jährlich betragen. Begründet werden diese finanziell aufwendigen Maßnahmen mit der Notwendigkeit des vorbeugenden Verbraucherschutzes bei gleichzeitigem Fehlen eines geeigneten Tests für spezifiziertes Risikomaterial.

Da bei der Herstellung von Lebensmitteln, insbesondere von Fleischerzeugnissen wie Brühwürsten, das Zusetzen von Hirngewebe auch technologische Vorteile wie bessere Emulgierbarkeit (Brüh- und Kochwurstbrät) und erhöhte Stabilität (Beefburger) bietet, ist es darüber hinaus zum Schutze des Verbrauchers notwendig, die mißbräuchliche Verwendung von zentralem Nervengewebe, insbesondere von Rückenmark und Hirngewebe, im Rahmen der Lebensmittelüberwachung zu kontrollieren.

Bislang werden zur Kontrolle auf unerlaubte Zusätze bei Fleischerzeugnissen histologische Präparate (10 µm Kryoschnitte, Färbung: Picroindigocarmin/Karmalaun) angefertigt, die von besonders geschulten Fachleuten lichtmikroskopisch untersucht werden. Mit diesem Verfahren können neben den zu erwartenden zulässigen Bestandteilen wie Muskulatur, Bindegewebe oder Gewürze, nicht zulässige Gewebe wie Niere, Pansen oder auch Haut identifiziert werden.

Es hat sich jedoch gezeigt, daß zentrales Nervengewebe in Fleischerzeugnissen mittels lebensmittelhistologischer Routinediagnostik nicht erfaßt werden kann. Weiterhin gilt dies auch für Ultradünnschnitte mit herkömmlicher Färbetechnik (Hämatoxylin/Eosin) sowie für Spezialfärbungen (Silberimprägnierung der Neurofibrillen, Markscheiden-, NISSL-Färbung). Auch sonstige charakteristische Bestandteile des zentralen Nervengewebes wie Hirnhäute oder Astrozyten sind mit Hilfe herkömmlicher Verfahren nicht identifizierbar. Offensichtlich werden diese Strukturen durch die bei der Herstellung von Fleischerzeugnissen verwendeten technologischen Verfahren (Homogenisierung, Erhitzung) weitgehend zerstört bzw. verändert und/oder maskiert.

Es besteht daher Bedarf an einem schnellen Testverfahren, mit dem der Zusatz an spezifiziertem Risikomaterial in Erzeugnissen auch noch in geringen Mengen nachgewiesen werden kann.

Vor kurzem ist im Hinblick auf die BSE ein solches Verfahren zum Nachweis von zentralem Nervengewebe in Fleischerzeugnissen beschrieben worden (E. Lücker und M. Bülte, Fleischwirtschaft 1997, 77: 836-840). Dabei wird mit einem enzymatisch-photometrischen Verfahren der Cholesteringehalt der Erzeugnisse bestimmt. Dabei ermöglicht die Erfassung der Erhöhung des Cholesteringehaltes durch zentrales Nervengewebe bei Brühwürsten den Nachweis von Zusätzen bereits ab 2% (statistische Sicherheit: 99,9%). Dieses Verfahren hat den Vorteil des geringen instrumentellen, Zeit- und Kostenaufwands. Es weist jedoch den Nachteil der geringen Spezifität auf. Interferenzen durch Zusätze an Ei, Eigelb, Leber oder Niere sowie 3-β-Hydroxysterine können nicht ausgeschlossen werden. Es kann daher in der Praxis der Lebensmittelüberwachung als schnelles Screeningverfahren zur Identifizierung von Verdachtsproben dienen.

Schließlich sind neuronenspezifische Proteine bekannt, die z.T. nahezu ausschließlich im Bereich des zentralen Nervensystem lokalisiert sind. Hierzu zählt die neuronenspezifische Enolase: NSE (D. Schmechel et al., Science, 1978, 199: 313-315) sowie das saure Gliafaserprotein: 'glial acidic protein', GFAP (D. Dahl und A. Bignami, in: A. Laitha: Handbook of Neurochemistry, Plenum Press, New York, 1983: 127 - 151).

Es wurde gezeigt, daß der Nachweis von NSE mittels monoklonaler Antikörper in Fleischerzeugnissen die Erfassung von Zusätzen an zentralem Nervengewebe ab 1% mit absoluter Spezifität bei Brühwürsten ermöglicht (Zitat: in dem noch nicht vorveröffentlichten Deutschen Patent Nr. 198 04 216). Allerdings interferiert der hohe Fettgehalt der Fleischerzeugnisse bei der Detektion derart, daß die weitgehende Entfernung der Fettmatrix bei diesem Verfahren unerläßliche Voraussetzung ist. So konnten ohne Fettextraktion bei Kochwürsten mit hohem Fettgehalt auch erheblich Zusätze an Hirngewebe nicht nachgewiesen werden. Bei Brühwürsten wurde der Nachweis von Zusätzen an Hirngewebe unter 1% erst nach Fettextraktion möglich.

L. F. Eng et al. (1976) Analytical Biochemistry 71, 243-259 offenbart einen immunoradiometrischen Assay zum Nachweis von Gehirn-spezifischen GFAP. Der Assay basiert auf der Reaktion der zu untersuchenden Probelösung mit einem auf einer Festphase immobilisierten anti-GFAP-Antikörper. Das auf diese Weise immobilisierte GFAP wird durch Reaktion mit einem radioaktiv-markierten sekundären anti-GFAP-Antikörper nachgewiesen. Die Methode kann zur Beobachtung der Verteilung von GFAP in verschiedenen Gewebe, Zellen und biologischen Flüssigkeiten in Abhängigkeit von Entwicklung, Alter und/oder bestimmten Erkrankungszuständen verwendet werden. M. Albrechtsen et al. (1985) Journal of Neurochemistry 44, 560-566 offenbart ein ELISA Assay zur Bestimmung von GFAP, das auf der Verwendung von spezifischen monoklonalen anti-GFAP-Antikörpern basiert.

Die Erfindung hat damit zum Ziel, den weiterhin bestehenden Bedarf an einem spezifischen, leicht durchzuführenden Verfahren zu befriedigen, so daß insbesondere zentrales Nervengewebe noch bei einem geringen Gehalt in Erzeugnissen ohne notwendige Fettextraktionsschritte zweifelsfrei nachgewiesen werden kann.

Diese Ziel wird nun mit dem erfindungsgemäßen Verfahren nach Anspruch 1 erreicht.

Es wurde nämlich gefunden, daß sich der Zusatz von zentralem Nervengewebe durch den Gehalt an GFAP nachweisen läßt, wobei der Gehalt direkt proportional zur zugesetzten Menge an zentralem Nervengewebe ist. Dies ist umso überraschender, weil umfangreiche Untersuchungen der Erfinder gezeigt haben, daß in erhitzten Fleischerzeugnissen wie Brüh- und Kochwürsten mit definierten Zusätzen an Gehirn bei der Polyacrylamid-Gelektrophorese mit Isoelektrischer Fokussierung (PAGIF) keine spezifischen Banden erhalten werden, die sich vom gleichen Produkt ohne Zusatz an Gehirn unterscheiden. Sowohl die Struktur als auch die physiko-chemischen Eigenschaften der ZNS-spezifischen Proteine werden offensichtlich infolge der erheblichen technologischen Einwirkungen (Homogenisierung sowie Erhitzung mindestens auf 80°C über etw 60 Minuten) nachhaltig beeinträchtigt. Dies gilt auch für den Nachweis von derartigen charakteristischen Proteinen mittels immunologischer Methoden.

Erfindungsgemäß wurde nun gezeigt, daß sich trotz der oben beschriebenen Proteindenaturierung ein spezifischer Nachweis von zentralem Nervengewebe in Erzeugnissen bzw. Präparaten durchführen läßt. Dieser Nachweis läßt sich mit saurem Gliafaserprotein (GFAP) durchführen. Bislang diente das GFAP ausschließlich als Marker in nativen, nicht erhitzten Geweben oder Körperflüssigkeiten im Rahmen der Grundlagenforschung (wie histologisch-embryologische Untersuchungen) und der klinischen Tumordiagnostik.

Überraschenderweise wurde festgestellt, daß GFAP quantitativ auch bei intensiv homogenisierten und erhitzten Erzeugnissen wie Brühwürsten nachweisbar bleibt. Auch bei sehr starker Verdünnung des ZNS in Fleischerzeugnissen (0,25% Hirnzusatz) wird eine deutliche Reaktion erhalten.

Überraschenderweise wurde weiterhin festgestellt, daß der Nachweis auch ohne vorherige Fettextraktion sowohl bei Brüh- als auch bei Kochwürsten von Zusätzen an Hirngewebe unter 1% selektiv erfolgt.

Die Erfindung betrifft auch einen Testkitt zur Durchführung des Verfahrens. Dabei wird ein an GFAP spezifisch bindender Antikörper mittels bekannten Verfahren an eine Festphase immobilisiert. Die zu untersuchende Probe wird dann mit dem vorzugsweise gelösten Protein in Kontakt gebracht und in einem geeigneten Puffersystem mittels der Antikörper an die Festphase gebunden. Nach Waschen des so erhaltenen immobilisierten Antikörper-GFAP-Komplexes wird dann ein weiterer, mit einem Marker versehener sekundärer Antikörper zugesetzt, der an ein anderes Epitop des GFAP bindet und die Menge des Markers bestimmt. Die Menge an gebundenem Marker ist direkt proportional der Menge an GFAP in der Probe. Zweckmäßigerweise enthält der Testkit Referenzmaterial mit unterschiedlichen und bekannten Gehalten an zentralem Nervengewebe zu Sicherung der Analysenqualität.

Erfindungsgemäß ist es auch möglich, die Analytkonzentration mittels Biosensoren zu bestimmen, wie z. B. amperometrische Sensoren, potentiometrische, ionenselektive potentiometrische oder photometrische Sensoren oder auch solche mittels Halbleiterelektroden wie Feldeffekttransistoren (FET), chemosensitive Feldeffekttransistoren (CHEMFET), suspended-gate-Feldeffekttransistoren (SGFET) oder ionensensitiven Feldeffekttransistoren. Derartige Biosensoren sind zusammenfassend in E. A. H. Hall und G. Hummel in "Biosensoren", Springer Verlag Heidelberg, Deutschland, 1995 beschrieben. Weitere Entwicklungen von ionensensitiven Feldeffekttransistoren (ISFET) oder optischen Detektoren sind unter anderem von F. Aberl und H. Wolf in "Aktuelle Trends in der Immunsensorik", Labor 2000, S. 70-96 (1993) beschrieben. Ebenfalls geeignet ist das erfindungsgemäße Verfahren für die Durchführung mittels piezoelektrischen Schwingquarzen und Oberflächenwellenelementen, welche als Mikrowaagen verwendet werden können. Dabei wird der primäre Antikörper (der sog. Catcher) auf einem piezoelektrischen Substrat immobilisiert und nach Bindung mit dem zu analysierendem GFAP gemessen. Derartige Sensoren sind beispielsweise von A. Leidl et al. in "Proceedings of the Second International Symposium on Minaturized Total Analyses Systems µTAS", Basel 1996, beschrieben. Quarzkristallmikrowaagen, wie sie von C. Köslinger et al., Fresenius J. Anal. Chem. (1994), 349: 349-354, beschrieben sind, haben sich als besonders geeignet erwiesen.

Die erfindungsgemäß zu verwendenden Antikörper sind auf an sich bekannte Weise erhältlich. Dabei werden zuerst Gliafaserproteine isoliert, wie beispielsweise bei M. Noppe et al. (In: H. Peeters (Hrsg.) Protides of the Biological Fluids, Pergamon Press, New York, 1979, 27: 813-816), bei Jeesen und Mirsky (Journal of Neuroimmunology, 1985, 8: 377-393) oder bei Eng und DeArmond (In: H. M. Zimmerman (Hrsg.) Progress in Neuropathology, Raven Press, New York, 1983,5: 19-39) beschrieben ist. Danach wird ein Versuchstier mit dem so erhaltenen Gliafaserprotein bzw. mit solchen Bruchstücken davon, welche die entsprechenden Epitope aufweisen, immunisiert und die so gebildeten Antikörper isoliert.

Vorzugsweise werden jedoch nach der Methode von Köhler und Willstein erhältliche monoklonale Antikörper verwendet. Dabei werden beispielsweise BALB/C-Mäuse mit gereinigtem Gliafaserprotein (Debus, Weber et al., Differentiation, 1983, 25: 193-203) immunisiert und die Milzzellen dieser Tiere mit einer Myelomazellinie, beispielsweise PA I, fusioniert. Die in den Ascites sezernierten Antikörper werden, beispielsweise im ELISA oder RIA, auf ihre Spezifität getestet und isoliert. Üblicherweise gehören die so erhaltenen Antikörper zur Klasse IgG1. Derart erhaltene Antikörper reagieren beispielsweise mit Astrocyten, mit Bergmann'schen Gliazellen und mit Tumoren der Neuroglia in Gefrier- und Paraffinschnitten. Derartige monoklonale Antikörper sind bereits im Handel erhältlich und werden beispielsweise von der Camon Laborservice GmbH, Wiesbaden, Deutschland (monoklonaler Antikörper E 008) und von Boehringer Mannheim GmbH, Deutschland (hergestellt aus den Maus-Maus Hybridklonen G-A-5) vertrieben.

### Beispiel 1:

Es wurden Brühwürste mit definierten Zusätzen an Rindergehirn hergestellt, wie dies bei Lücker und Bülte (Fleischwirtschaft 1997, 77: 836-840) beschrieben ist. Bei diesen Brühwurststandards lag der Anteil an Hirnzusatz zwischen 1 und 33,3%. Zur Kontrolle wurde ein gleichartiger Brühwurststandard ohne Hirnzusatz sowie ein Brühwurststandard ohne Hirnzusatz jedoch mit Zusatz an 16% Eigelb verwendet (s. Tabelle). Die Proben wurden unter Kühlung im Tris-Harnstoffpuffer im Potter extrahiert (Extraktionspuffer: TRIS-Harnstoff-Puffer aus 20 mM Tris (Hydroxymethyl)-Aminomethan, 8 M Harnstoff, pH 7,6) und 15 Minuten bei 20.000 UpM zentrifugiert und zweifach filtriert. Das Filtrat wurde im Verhältnis 50:1 mit Probenpuffer (Tris-SDS-Mercaptoethanol-Harnstoff, Probenpuffer zu gleichen Teilen aus Sammelgelpuffer, 10%-igem Gew./Vol. 2 Mercaptoethanol, 10 %igem Gew./Vol. SDS und 8 M Harnstoff; Sammelgelpuffer: 0,5 Tris (Hydroxymethyl)-Amminomethan, 1% Gew./Vol. SDS) verdünnt. Diese Probenextrakte können bei -18 °C über mehrere Monate hindurch ohne Qualitätsverluste gelagert werden.

Die Proteinkonzentration der Extrakte wurde mit einem Proteintest von Bio-Rad (Richmond, USA) bestimmt. Die Proteine wurden mittels einer SDS-Gel-Elektrophorese mit 10%igen Acrylamidgelen nach Laemmli (Nature 1970, 227: 680-685) aufgetrennt. Dabei wurde bei jeder Probe 10 - 100 µg Protein aufgetragen. Nach der Auftrennung wurde z. T. mit Coomassie-Blau angefärbt. Als Marker diente der Molekularmasse-Kalibrierungskit LMW von Pharmacia (Uppsala, Schweden).

Die aufgetrennten extrahierten Proteine wurden dann im Elektroblotverfahren (CTI, Idstein/Taunus) auf Nitrocellulose-Membranen (Optitran BA-S85, Schleicher und Schuell) bzw. auf PVDF-Membranen (Immobilon-P, Millipore, Bedford, USA) übertragen und mittels primärer monoklonaler Antikörper in Verbindung mit sekundären Antikörpern markiert, die mit Peroxidase konjugiert waren (direkt bzw. biotyniliert). Als Substrat wurde 3,3'-Diaminobenzidin-tetrahydrochloridhydrat (DAB, Aldrich, Milwaukee, USA) verwendet.

Als Antikörper wurden kommerziell erhältliche, monoklonale, gegen humanes GFAP gerichtete Antikörper aus Maus-Maus-Hybridzellen des Klons MIG-G2 (CAMON, Wiesbaden) sowie G-5-A (Boehringer, Mannheim) verwendet.

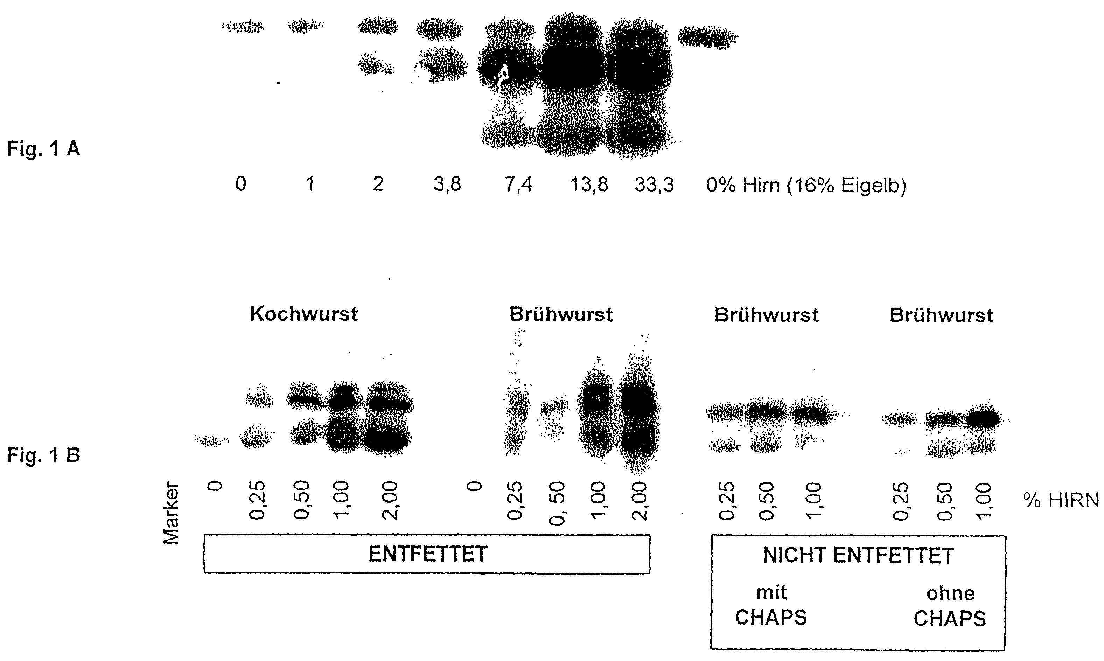

Im Immunoblot sind bis zu 4 Banden im Bereich um 50 kD erkennbar (s. Fig. 1 A: Immunoblot von Brühwürsten mit 1 bis 33,3% Hirnzusatz, sowie Zusatz an Eigelb (16%) nach Fettextraktion, Anti-GFAP (Camon)). Die Ergebnisse für die spezifische Bande mit der höchsten Intensität sind der Tabelle 1 zu entnehmen.

### Beispiel 2:

Beispiel 1 wurde mit den genannten Brühwurststandards wiederholt. Zusätzlich wurden Brühwurststandards mit 0, 0,25, 0,5, 1,0 und 2,0% Zusatz an Rinderhirn sowie Kochwurststandards mit Hirnzusätzen von 0 bis 32 % verwendet. Die Proben wurden wie unter Beispiel 1 direkt, unter Zusatz von CHAPS (3-[(3-Cholamidopropyl)dimethylammonio]-1-propansulfonat, 10 mM, Sigma, Deisenhofen) sowie nach Verminderung des Fettgehalts extrahiert. Zur Verminderung des Fettgehalts wurden die Proben manuell mit einem Messer fein zerkleinert. Davon wurden 10 g auf einem Faltenfilter (Nr. 597½, Schleicher und Schuell, Dassel) eingewogen, in eine Extraktionshülse aus Filterpapier (Nr. 603, Schleicher und Schuell, Dassel) eingebracht und mit Watte verschlossen. Die Extraktionshülse mit Probe wurde darauf in einem Extraktionsapparat nach Soxhlet mit Petroleum-Benzin (1,5-fache des Volumens des Extraktionsaufsatzes) acht Stunden unter Rückfluß extrahiert. Nach Rückdestillierung des Lösungsmittels wurden die Extraktionshülsen entnommen und und bei Raumtemperatur 1 Stunde getrocknet. Die Reduktion des Fettgehaltes der so behandelten Proben lag bezogen auf den ursprünglichen Fettgehalt der Probe zwischen 18 und 53%. Die Ergebnisse sind in der Fig. 1 B (Immunoblot von Brüh- und Kochwürsten mit 0,25 bis 2,0% Hirnzusatz nach unterschiedlichen Extraktionen, Anti-GFAP (Boehringer)) und der Tabelle 1 zusammengestellt.

**Tabelle 1.**

| **Auflistung der Ergebnisse (semiquantitativ) aus Beispiel 1 (ohne Fettextraktion) und Beispiel 2 (mit Fettextraktion) für die spezifische GFAP-Bande mit hoher Intensität.** | | | | |
|---|---|---|---|---|
| **Probe** | **Hirn-Zusatz (%)** | **Sonst. Zusatz (%)** | **Extraktion** | **Response**^{**1**} |
| Brühwurst | 0 | Ohne | Ohne Fettextraktion | - |
| | 1 | | | ++ |
| | 2 | | | ++ |
| | 3,8 | | | +++ |
| | 7,4 | | | +++ |
| | 13,8 | | | +++ |
| | 33,3 | | | +++ |
| | 0 | Eigelb, 16% | | - |
| | 0 | Ohne | Mit Fettextraktion | - |
| | 0.25 | | | + |
| | 0,5 | | | + |
| | 1 | | | ++ |
| | 2 | | | ++ |
| Kochwurst | 0 | Leber, 16% bzw. Leber, 32% | Ohne Fettextraktion | - |
| | 1 | | | ++ |
| | 2 | | | ++ |
| | 4 | | | +++ |
| | 8 | | | +++ |
| | 16 | | | +++ |
| | 32 | | | +++ |
| | 0,25 | | Mit Fettextraktion | + |
| | 0,5 | | | + |
| | 1 | | | + |
| | 2 | | | ++ |
| | 4 | | | +++ |

| | | | | |
|---|---|---|---|---|
| ¹ - Bande nicht erkennbar + Bande (mit bloßem Auge) erkennbar ++ Bande gut erkennbar +++ Bande sehr stark ausgeprägt* * Einschließlich noch stärker ausgeprägten Banden: diese werden hier nicht quantifiziert. | | | | |

## Patentansprüche

1. Verfahren zum qualitativen und quantitativen Nachweis von zentralem Nervengewebe in erhitzten Fleischerzeugnissen, **dadurch gekennzeichnet, dass** man zugesetztes Gehirn oder Rückenmark durch den Gehalt an saurem Gliafaserprotein (GFAP) und Bruchstücken davon in einer zu untersuchenden Probe mittels eines an einer Festphase immobilisierten anti-GFAP-Antikörpers erfasst.

2. Verfahren nach Anspruch 1 **dadurch gekennzeichnet, dass** der Antikörper ein monoklonaler Antikörper ist, der aus der Hybridoma-Zellinie MIG-G2 und/oder G-A-5 erhältlich ist.

3. Testkit zum Nachweis von Zusätzen an zentralem Nervengewebe in erhitzten Fleischerzeugnissen, **dadurch gekennzeichnet, dass** er; einen an der Restphase gebundenen Antikörper gegen GFAP sowie für die analytische Qualitätssicherung Referenzmaterial mit bekannten Gehalten an zentralem Nervengewebe enthält.

4. Testkit nach Anspruch 3, **dadurch gekennzeichnet, dass** er einen markierten Antikörper enthält.

## Claims

1. Method of qualitatively and quantitatively detecting central nerve tissue in heated meat products, **characterised in that** added brain or spinal cord is detected by the content of glial fibrous acidic protein (GFAP) and fractions thereof in a sample which is to be tested by means of an anti-GFAP antibody immobilised on a solid phase.

2. Method according to claim 1, **characterised in that** the antibody is a monoclonal antibody which is obtainable from the hybridoma cell line MIG-G2 and/or G-A-5.

3. Test kit for detecting additions to central nerve tissue in heated meat products, **characterised in that** it includes an antibody against GFAP, which is combined with the solid phase, as well as including, for the analytical guarantee of quality, reference material with known contents of central nerve tissue.

4. Test kit according to claim 3, **characterised in that** it includes a reference antibody.

## Revendications

1. Procédé de détection qualitative et quantitative de tissu du système nerveux central dans des produits carnés chauffés, **caractérisé en ce que** l'on détermine la présence de cervelle ou de moelle épinière par la teneur en protéines glio-fibrillaires acides (GFAP) et de parties pontées de celles-ci dans un échantillon à examiner à l'aide d'un anticorps anti-GFAP immobilisé sur une phase solide.

2. Procédé suivant la revendication 1, **caractérisé en ce que** l'anticorps est un anticorps monoclonal, qui est obtenu à partir de la lignée cellulaire d'hybridome MIG-G2 et/ou G-A-5.

3. Kit d'épreuve pour détecter des additions de tissu du système nerveux central dans des produits carnés chauffés, **caractérisé en ce qu'**il contient un anticorps contre la GFAP, lié sur la phase solide, ainsi qu'un matériau de référence avec teneur connue en tissu du système nerveux central pour la détermination analytique.

4. Kit d'épreuve suivant la revendication 3, **caractérisé en ce qu'**il contient un anticorps marqué.
